Europäisches Patentamt

European Patent Office

Office européen des brevets

(19)

(11) Veröffentlichungsnummer: **0 326 145**
**A1**

## (12) EUROPÄISCHE PATENTANMELDUNG

(21) Anmeldenummer: 89101409.4

(22) Anmeldetag: 27.01.89

(51) Int. Cl.⁴: **A61K 31/505** , **A61K 9/50** , **A61K 47/00** , **A61L 25/00** , **A61L 15/03**

(30) Priorität: 29.01.88 DE 3802654
18.08.88 DE 3828044

(43) Veröffentlichungstag der Anmeldung:
02.08.89 Patentblatt 89/31

(84) Benannte Vertragsstaaten:
ES GR

(71) Anmelder: **Minninger, Konrad**
**Hattinger Strasse 45**
**D-5830 Schwelm(DE)**

(72) Erfinder: **Minninger, Konrad**
**Hattinger Strasse 45**
**D-5830 Schwelm(DE)**
Erfinder: **Tang, David, Dr.**
**Hauptstrasse 304**
**D-4690 Herne 2(DE)**
Erfinder: **Oberhagemann, Rainer**
**Hauptstrasse 304**
**D-4690 Herne 2(DE)**

(74) Vertreter: **Redies, Bernd, Dr. rer. nat. et al**
**COHAUSZ & FLORACK Patentanwaltsbüro**
**Schumannstrasse 97 Postfach 14 01 20**
**D-4000 Düsseldorf 1(DE)**

(54) **Silbersulfadiazinhaltiges Mittel zur lokalen äusserlichen Therapie.**

(57) Das silbersulfadiazinhaltige Mittel zur lokalen Therapie von Herpes labialis, Herpes genitalis, Herpes corporis, Zoster, Windpocken, Ekzema herpeticatum und Verbrennungen II. und III. Grades im Bereich der Humanmedizin enthält 0,01 Gew.-% bis 10 Gew.-% Silbersulfadiazin, 0,01 Gew.-% bis 10 Gew.-% eines mehrwertigen Alkohols, 0,01 Gew.-% bis 10 Gew.-% eines Lokalanästheticums, 70 Gew.-% bis 99,9 Gew.-% GEL-Präparation jeweils bezogen auf das Gesamtgewicht des Mittels = 100.

EP 0 326 145 A1

## Silbersulfadiazinhaltiges Mittel zur lokalen äußerlichen Therapie

Die Erfindung betrifft ein silbersulfadiazinhaltiges Mittel zur lokalen, äußerlichen Therapie von Herpes labialis, Herpes genitalis, Herpes corporis, Zoster (Herpes zoster), Windpokken, Ekzema herpeticatum sowie Verbrennungen II. Und III.Grades im Bereich der Humanmedizin

Es ist beispielsweise aus der Veröffentlichung von Reinhard Zimmermann und Wolfgang Kräuter, Offenbach/M. in der Zeitschrift "Krankenhauspharmazie", 7. Jahrgang Nr. 11, 1986, bekannt, daß Silbersulfadiazin ein Nicht-Toxisches Oberflächenantiseptikum ist, das in einer Cer-haltigen Creme äußerlich zur Vorbeugung und Behandlung von Wundinfektionen nach Verbrennungen, eingesetzt wird.

Desweiteren ist Silbersulfadiazin auch in einer mit Wasser mischbaren Softcreme zur Behandlung von Herpes zoster vorgeschlagen worden, vlg. die Veröffentlichung von L.F.Montes, G. Muchinik, Ch. L. Fox, "Response of Varicella Zoster Virus und Herpes Zoster to Silver Sulfadiazine"; Cutis, Vol.38 Dez. 1986, S. 363 - 365.

Dazu ist eine vielfach wiederholte Reinigung der befallenen Hautstellen mit einer aufweichenden Inertseife und nachfolgende Auftragung der Silbersulfadiazin enthaltenden Softcreme erforderlich.

Silbersulfadiazin wird in verschiedenen Kombinationen verwendet .um sekundäre bakterielle Infektionen zu verhindern und einen synergistisch bakteriostatischen Effekt zu erreichen.

Im Gebrauch der bekannten silbersulfadiazinhaltigen Mittel hat sich herausgestellt, daß diese bekannten Mittel dadurch, daß sie als Cremes aufgetragen werden, die für den Erfolg der Behandlung in der ersten Phase des Heilprozesses so wichtige rasche Austrocknung der betroffenen Hautstellen bzw. Wundbereiche, nicht zulassen.

Weil eine Creme einen gewissen Fettanteil enthält, wird z.B. bei großflächigem Zoster ein mehrmaliges Abwaschen notwendig. Dieses belastet die Patienten sehr stark.

Die Aufgabe der Erfindung besteht darin, ein silbersulfadiazinhaltiges Mittel der eingangs genannten Art zu schaffen, das bei guter Verträglichkeit und leichter Aufbringung eine rasche Austrocknung der Wundbereiche während der ersten Phase des Heilprozesses ohne nachteilige Belastung für die Patienten ermöglicht.

Erfindungsgemäß wird diese Aufgabe dadurch gelöst, daß das Mittel 0, o1 Gew.% bis 10 Gew.% Silbersulfadiazin in 99,9 Gew.% bis 90,0 Gew. % einer wäßrigen Gelpräparation enthält.

Es hat sich gezeigt, daß das erfindungsgemäß zusammengesetzte Mittel durch die Gelpräparation eine rasche Austrocknung der Wundbereiche bereits in der ersten Phase des Heilprozesses sicherstellt, ohne daß die Wirkung des Silbersulfadiazins dadurch beeinträchtigt wird. Die Anwendung des Mittels ist dadurch unter Bedingungen möglich, die praktisch keine Belastung für den Patienten darstellen.

Vorteilhafterweise enthält das erfindungsgemäße Mittel bei Bedarf (z.B. großflächigem, schmerzhaften Zoster) zusätzlich ein Lokalanästhetikum.

Ausführungsbeispiele der Erfindung werden nachstehend an Hand einiger Rezepturen erläutert:

Alle eingesetzten Stoffe genügen den Anforderungen des DAB.

Als Gelpräparation sind eine Vielfalt von auf dem Markt befindlichen Gelpräparationen, geschmacks und geruchsneutraler Art, geeignet.

Dafür sind auch sogenannte Liposomen siehe Veröffentlichung W.Raab "Liposomen- eine neue Form dermatologischer Wirkstoffträger" in der Zeitschrift Ärztliche Kosmetologie 18, 213 -224 (1988), geeignet.

Beispiel für eine Gelpräparation ist die nachfolgend wiedergegebene Gelzusammensetzung:

| Polyacrylsäure | 0,5 gramm |
|---|---|
| Isopropanol | 5,0 gramm |
| Propylenglykol | 20,0 gramm |
| Natriumhydroxidlösung | 1,2 gramm |
| Wasser | 73,3 gramm |
| | 100,- gramm |

In einem solchen Gel kann das Silbersulfadiazin mit üblichen Mitteln entweder direkt oder nach vorheriger Auflösung in einem mehrwertigen Alkohol wie Glyzerin, Propylenglykol oder dergleichen verteilt werden.

Die so mit der vorgenannten Gelpräparation erhältlichen Mittel haben allgemein die folgende Zusammensetzung in Gew.%, jeweils bezogen auf das Gesamtgewicht = 100.

| Silbersulfadiazin | 0,01 | Gew.% bis insges. | 10,0 | Gew.% |
|---|---|---|---|---|
| Glyzerin | 0,01 | Gew.% bis insges. | 10,0 | Gew.% |
| GEL | 99,98 | Gew.% bis insges. | 80,0 | Gew.% |

Für die Anwendung hat es sich in vielen Fällen als zweckmäßig erwiesen, dem Mittel ein Lokalanästhetikum zuzusetzen. Dafür sind viele bekannte Lokalanästhetika geeignet, z.B. Bupivacainhydrochlorid. Die so modifizierten Mittel haben die folgende Zusammensetzung in Gew.% jeweis bezogen auf das Gesamtgewicht = 100:

| Silbersulfadiazin | 0,01 | Gew.% bis insges. | 10,0 | Gew.% |
|---|---|---|---|---|
| Glyzerin | 0,01 | Gew.% bis insges. | 10,0 | Gew.% |
| Bupivacain HCl | 0,01 | Gew.% bis insges. | 10,0 | Gew.% |
| GEL | 99,97 | Gew.% bis insges. | 70,0 | Gew.% |

Die erfindungsgemäßen Mittel sind auch bei längerer Anwendung gut verträglich; bisher wurden keine Nebenwirkungen oder allergische Reaktionen beobachtet. Dabei wird durch die Austrocknung, die das Gel schon in der ersten Phase des Heilprozesses bewirkt, eine hohe Wirksamkeit bereits nach sehr kurzer Zeit erreicht, die antivirale Wirkung und die ausgeprägte antibakterielle Wirkung des Wirkstoffes Silbersulfadiazin auf Herpesviren bzw. vor allem gegen Pseudomonas kommt voll zur Geltung, um sekundäre bakterielle Infektionen zu verhindern und zu behandeln. Die langanhaltende Schmerzfreiheit durch das verwendete Lokalanästhetikum ist ein äußerst hervorzuhebender Vorteil, da alle Erkrankungen vorgenannter Art teilweise äußerst schmerzhaft sind.

Weiterhin hat sich herausgestellt, daß die vorstehend zusammengesetzten Mittel nicht sehr lichtempfindlich sind, sodaß für die Herstellung, Aufbewahrung und Anwendung dieser Silbersulfadiazin enthaltenden Mittel keine besonderen Lichtschutzmaßnahmen getroffen werden müssen.

Besonders bevorzugte Mittel sind wir folgt zusammengesetzt, jeweils in Gew.% bezogen auf das Gesamtgewicht = 100

| Silbersulfadiazin | 2 Gew.% |
|---|---|
| Glyzerin | 2 Gew.% |
| Bupivacain HCl | 2 Gew.% |
| Gel | 94 Gew.% |

| Silbersulfadiazin | 4 Gew.% |
|---|---|
| Propylenglykol | 4 Gew.% |
| Bupivacain HCl | 2 Gew.% |
| Gel | 90 Gew.% |

Die therapeutische Anwendung eines Ausführungsbeispiels, das 2 Gew.% Silbersulfadiazin, 2 Gew.% Glyzerin, 96 Gew.% einer Gelpräparation enthält, wird nachfolgend an Hand einer Patientengruppe von 55 Personen erläutert,

Erkrankung:

a) Zoster (Herpes zoster)

Therapeutische Anwendung lokal äußerlich, 2 - 3 Anwendungen pro Tag
nach 1 - 3 Tagen deutliche Eintrocknung der Bläschen ohne bakterielle Sekundär-Infektionen. Keine neue Bläschenbildung.
Nach 5 - 14 Tagen waren bei allen Patienten die Bläschen eingetrocknet.

EP 0 326 145 A1

Bei jüngeren Patienten bis 30 Jahre u.a. durch stark verkürzten Krankheitsverlauf keine oder nur geringe postzosterische Neuritis bzw. Neuralgie.

Bei älteren Patienten ab 60 Jahren, die früh- bzw. rechtzeitig behandelt wurden und die keine zu starke Ausdehnung der Bläschen hatten, kam es zu keiner oder nur zu geringer post-zosterischer Neuralgie

Bei älteren Patienten ab 60 Jahren oder Patienten mit großflächigem, teilweise verschlepptem Zoster wurde vorgenanntes Mittel durch ein Lokalanästhetikum (2 Gew.-%) ergänzt. verkürzter Krankheitsverlauf erreicht, geringere post-zosterische Neurits bzw. Neuralgie, keine bakteriellen Sekundär-Infektionen.

Die Dauer des sonst üblichen Krankheitsverlaufes konnte in allen Fällen um bis zu einem Drittel verkürzt werden.

b) Herpes labialis

Therapeutische Anwendung lokal äußerlich, 2 - 3 Anwendungen pro Tag

Nach 1 - 3 Tagen deutliche Eintrocknung der Bläschen ohne bakterielle Sekundär-Infektion, keine neue Bläschenbildung.

Nach 3 - 5 Tagen waren bei allen Patienten die Bläschen eingetrocknet.

Die Dauer des sonst üblichen Krankheitsverluafes konnte in allen Fällen um mindestens ein Drittel verkürzt werden.

c) Herpes genitalis

Durch die Besonderheit der Gelpräparation, denn ein Gel wirkt im intertriginösen Raum (Haut auf Haut) besonders günstig, ein hervorzuhebender kurzer Krankheitsverlauf.

Nach 1 bis 3 Tagen deutliche Eintrocknung de Bläschen ohne bakterielle Sekundär-Infektion.

Nach 3 bis 5 Tagen waren bei allen Patienten die Bläschen eingetrocknet.

Die Dauer des sonst üblichen Krankheitsverlaufes konnte in allen Fällen um mindestens ein Drittel verkürzt werden.

Bei allen bisher beschriebenen Krankheitsbildern und deren Therapie mit Silbersulfadiazin-Gel sind folgende Factoren hervorzuheben:

1.) der kühlende und austrocknende Effekt des Silbersulfadiazin-Gels,

2.) die leichte Anwendbarkeit und die gute Kosmetik,

3.) die hohe Akzeptanz der Patienten durch die in Punkt 1 und Punkt 2 genannten Eigenschaften.

**Ansprüche**

1. Silbersulfadiazin enthaltendes Mittel zur lokalen äußerlichen Therapie von Herpes labialis, Herpes genitalis, Herpes corporis, Zoster (Herpes zoster) Windpocken, Ekzema herpeticatum und Verbrennungen II. und III. Grades, dadurch gekennzeichnet, daß das Mittel 0,01 Gew.-% bis 10,0 Gew.-% Silbersulfadiazin und 99,9 Gew.-% bis 90,0 Gew.-% einer Gelpräparation enthält, jeweils bezogen auf das Gesamtgewicht des Mittels = 100.

2. Mittel nach Anspruch 1, dadurch gekennzeichnet, daß die Gelpräparation ein Gel auf der Basis eines Polyacrylsäuresalzes ist, das ein- und mehrwertige Alkohole enthält.

3. Mittel nach Anspruch 1, dadurch gekennzeichnet, daß es sich um ein Gel auf Basis von Liposomen, eine Liposomen-Gelpräparation handelt.

4. Mittel nach Anspruch 1 oder 2, dadurch gekennzeichnet, daß das Mittel 2 Gew.-% bis 4 Gew.-% Silbersulfadiazin, 2 Gew.-% bis 4 Gew.-% eines mehrwertigen Alkohols und 92 Gew.-% bis 96 Gew.-% der Gelpräparation enthält, jeweils bezogen auf das Gesamtgewicht des Mittels = 100.

5. Mittel nach Anspruch 4, dadurch gekennzeichnet, daß der mehrwertige Alkohol aus Glyzerin und/oder Propylenglykol ausgewählt ist.

6. Mittel nach einem der vorstehenden Ansprüche, dadurch gekennzeichnet, daß darin ein Lokalanästhetikum enthalten ist.

7. Mittel nach Anspruch 6, dadurch gekennzeichnet, daß der Anteil des Lokalanästhetikums 0,01 Gew.-% bis 10 Gew.-% beträgt, bezogen auf das Gesamtgewicht des Mittels = 100.

4

8. Mittel nach Anspruch 7, dadurch gekennzeichnet, daß der Anteil des Lokalanästhetikums 1 Gew.-% bis 3 Gew.-% beträgt, bezogen auf das Gesamtgewicht des Mittels = 100.

9. Mittel nach einem der Ansprüche 6 bis 8, dadurch gekennzeichnet, daß das Lokalanästhetikum Bupivacainhydrochlorid ist.

# EUROPÄISCHER RECHERCHENBERICHT

**Europäisches Patentamt**

Nummer der Anmeldung

EP 89 10 1409

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | US-A-4 376 764 (I.R. SCHMOLKA)<br>* Spalte 3, Zeilen 13-24; Spalte 3,<br>Zeilen 59-66; Ansprüche 1,5 *<br>--- | 1-9 | A 61 K 31/505<br>A 61 K 9/50<br>A 61 K 47/00<br>A 61 L 25/00<br>A 61 L 15/03 |
| X | US-A-4 659 700 (D.S. JACKSON)<br>* Spalte 1, Zeilen 37-44; Spalte 2,<br>Zeilen 3-4; Ansprüche *<br>--- | 1,4-5 | |
| D,A | CUTIS, Band 38, Dezember 1986, Seiten<br>363-365; L.F. MONTES et al.: "Response<br>of varicella zoster virus and herpes<br>zoster to silver sulfadiazine"<br>* Seite 364, Spalte 2, Zeilen 4-7 *<br>--- | 1-9 | |
| X | NL-A-7 408 580 (PHILIPS)<br>* Seite 3, Zeilen 19-31; Seite 4,<br>Zeilen 22-32; Ansprüche *<br>--- | 1 | |
| X | PHARMACEUTISCH WEEKBLAD, Band 118, Nr.<br>9, 1983, 170-174; R.H. BRIEDE:<br>"Toepassingen van carbomeerwatergel 1%"<br>* Seite 172, Spalte 2 *<br>--- | 1-9 | RECHERCHIERTE SACHGEBIETE (Int. Cl.4) |
| Y | FR-A-2 285 896 (NIPPON KAYAKU K.K.)<br>* Ansprüche 1-4,6; Seite 3, Zeilen<br>18-27 *<br>------ | 1-9 | A 61 K<br>A 61 L |

Der vorliegende Recherchenbericht wurde für alle Patentansprüche erstellt

| Recherchenort | Abschlußdatum der Recherche | Prüfer |
|---|---|---|
| DEN HAAG | 08-05-1989 | BERTE M.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)